# EUROPEAN PATENT APPLICATION

(11) **EP 4 105 225 A1**
(43) Date of publication of application: **21.12.2022**
(21) Application number: 21382539.1
(22) Date of filing: 18.06.2021
(51) Int. Cl.: C07K 7/02, C07K 14/005, G01N 33/569

(54) **PEPTIDE-BASED ASSAY TO DIFFERENTIATE ANIMALS INFECTED WITH CSFV FROM VACCINATED ANIMALS**

(71) Applicant: Institut de Recerca i Tecnologia Agroalimentàries, 08140 Caldes De Montbui (ES); Universitat Pompeu Fabra, 08002 Barcelona (ES); The United States of America, as Represented by the Secretary of Agriculture, Washington DC (US), Beltsville, MD 20705-5131 (US)
(72) Inventor: Ganges Espinosa, Llilianne, 08140 Caldes de Montbui, Barcelona (ES); Bohórquez Garzón, Jose Alejandro, 08140 Caldes de Montbui, Barcelona (ES); Andreu Martinez, David, 08002 Barcelona (ES); Defaus Fornaguera, Sira, 08002 Barcelona (ES); Gladue, Douglas, Beltsville, Maryland, 20705-5131 (US); Borca, Manuel V., Beltsville, Maryland, 20705-5131 (US)
(74) Representative: Clarke Modet & Co.

(57) **Abstract**

The present patent application discloses a dendrimeric peptide construct and method to differentiate animals infected with the Classical Swine Fever Virus (CSFV) from animals vaccinated with the CSFV FlagT4G vaccine.

The method disclosed herein comprises conducting two immunoassays, preferably ELISAs, to differentiate infected from vaccinated animals (DIVA); one immunoassay to detect humoral response against the dendrimeric peptide of the invention and a second immunoassay to detect humoral response against the E2 glycoprotein of the CSFV. Accordingly, the presently disclosed method allows the differentiation of three groups of individuals: animals vaccinated with FlagT4G vaccine, animals infected with CSFV and animals that are neither infected nor vaccinated.

## Description

### Technical field

This application relates to a peptide and method for the differentiation of animals infected with Classical Swine Fever Virus (CSFV) from animals vaccinated with the FlagT4G vaccine against CSFV.

### Background art

Classical swine fever (CSF) is a highly impactful disease for the pig industry throughout the world. CSF affects both domestic pigs and wild boars and remains endemic in Asia, areas of Central and South America and in many Eastern European countries [1]. The disease is caused by the CSF virus (CSFV), which belongs to the Pestivirus genus of the Flaviviridae family. The virus is composed of a lipid envelope, a capsid and a single stranded, positive-sense RNA genome of 12.3kb [2]. The genome carries a single long open reading frame (ORF) flanked by a 5'- and a 3'-untranslated regions. The ORF encodes four structural and eight non-structural proteins. The envelope glycoprotein E2 represents the major immunogenic protein of pestiviruses, plays a central role in virus entry and is associated with virulence. Besides E2, there are other proteins, such as the E^{rns} and the NS3, which are able to elicit immune response in infected animals, although at a lesser degree [2].

Control of CSFV in endemic countries relies on vaccination, mainly using live attenuated vaccines. These type of vaccines present several advantages that facilitate their use in developing countries, inducing high neutralizing antibodies titers at a short time after vaccination, conferring protection against highly virulent CSFV strains [3]. Nevertheless, these type of vaccines do not allow the differentiation of vaccinated from infected animals (DIVA concept) by using a serological test and thus cannot be used in countries where the disease has been or is in the process of being eradicated. Thus, there is not an effective vaccine (and accompanying diagnostic test) that fulfills the requirements necessary for a CSFV vaccine, in terms of protection and compliance with the DIVA concept [4].

Recently, a live attenuated vaccine prototype (named FlagT4G), based on a recombinant CSFV modified by progressively mutating a portion of the E2 gene of the highly pathogenic strain Brescia, has been developed [5,6]. More specifically, the T829**AVSPT**TLR837 epitope of the CSFV E2 glycoprotein has been mutated and replaced by the T**SFNMD**TLR amino acid sequence. Besides this, the virus also has a synthetic Flag^{®} insertion in the E1 glycoprotein. This vaccine has proven effective in inducing a sterilizing immune response as early as 3 days after vaccination, making it a highly promising vaccine prototype with DIVA potential [7]. However, the use of FlagT4G as a DIVA vaccine relies on its use with an accompanying DIVA diagnostic test, which has not been developed so far.

### Summary

The present patent application relates to a dendrimeric peptide construct and method for the differentiation of animals infected with Classical Swine Fever Virus (CSFV) from animals vaccinated with the FlagT4G vaccine against CSFV.

In one main embodiment of the present patent application it is disclosed herein a dendrimeric peptide construct (Lin₂NS3) comprising two copies of the natural CSFV-E2 epitope of SEQ ID NO: 1 linked to one copy of the epitope CSFV-NS3 of SEQ ID NO: 2. Said dendrimeric peptide construct is preferably according to formula I:

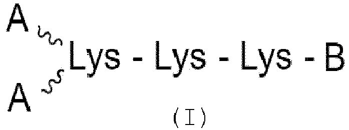

Wherein A is the CSFV-E2 epitope of SEQ ID NO: 1 and B is the CSFV-NS3 epitope of SEQ ID NO: 2.

In one embodiment, the dendrimeric peptide construct (formula I) is for use in an immunoassay for the detection of humoral response against Classical Swine Fever Virus (CSFV).

In one preferable embodiment, the dendrimeric peptide construct (formula I) is for use in an immunoassay for the detection of humoral response against CSFV as part of a method to differentiate infected animals from vaccinated animals.

In one preferable embodiment, the dendrimeric peptide construct (formula I) is for use in an immunoassay for the detection of humoral response against CSFV as part of a method to differentiate infected animals from animals vaccinated with FlagT4G.

As another main embodiment of the present patent application it is disclosed herein an *in vitro* method to differentiate animals infected with Classical Swine Fever Virus (CSFV) from animals vaccinated with FlagT4G, which comprises the following steps:
- Conducting an immunoassay, in a blood sample from the animal, for the detection of humoral response against the E2 glycoprotein of the CSFV;
- Conducting an immunoassay, in a blood sample from the animal, for the detection of humoral response against the dendrimeric peptide construct of the invention (formula I);
   wherein having a positive result in the first immunoassay while having a negative result in the second immunoassay is indicative that said animal has been vaccinated with FlagT4G.

In one embodiment the immunoassay is selected from ELISA, radioimmunoprecipitation assay, immunofluorescence assay enzyme immunoassay (EIA), radioimmunoassay (RIA), fluoroimmunoassay (FIA), chemiluminescent immunoassay (CLIA), counting immunoassay (CIA), immunoenzymometric assay (IEMA), immunochromatographic assay, a lateral flow immunoassay, a sandwich immunoassay, an immuno-PCR assay, a proximity ligation assay, a western blot assay, or an immunoprecipitation assay.

In a preferable embodiment, the diagnostic method is an ELISA.

The present application also describes an ELISA kit for the differentiation of animals infected with CSFV from animals vaccinated with FlagT4G vaccine, wherein said ELISA kit comprises ELISA microplates coated with the dendrimeric peptide construct of the invention and ELISA microplates coated with the CSFV E2 glycoprotein.

In one embodiment, the ELISA kit further comprises a sample diluent solution, a washing solution, an anti-pig IgG peroxidase, a revealing solution (3,3',5,5'-Tetramethylbenzidine (TMB)) and a stop solution (H₂SO₄ 1N).

### Detailed Description

This application relates to an *in vitro* method for the differentiation of animals infected with Classical Swine Fever Virus (CSFV) from animals vaccinated with the FlagT4G vaccine against CSFV. The application further discloses a dendrimeric peptide construct Lin₂NS3, for use in an immunoassay, preferably an ELISA assay, for supporting the DIVA (differentiation of infected from vaccinated animals) diagnosis of animals vaccinated with the FlagT4G vaccine against Classical swine fever virus.

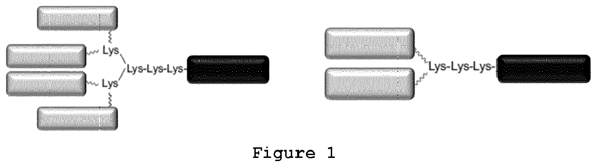

The dendrimeric peptide constructs described herein are powerful tools for the detection of CSFV. More specifically, it is important to emphasize that the dendrimeric constructs bearing two copies of a peptide attached to one copy of a different peptide (referred to as a 2:1 ratio) are more efficient, in detecting antibody response, than those bearing the same peptides but using four copies of the first peptide, instead of 2 (referred to as a 4:1 ratio, Figure 1).

In addition, immunoassays performed with the dendrimeric peptide construct of the present application showed no detection for animals vaccinated with the FlagT4G vaccine.

Considering this, the present patent application discloses a DIVA diagnostic method for the FlagT4G vaccine, taking into account the mutated epitope within the E2 glycoprotein of the vaccine and using the dendrimeric strategy for antigen presentation.

The *in vitro* method to differentiate animals infected with Classical Swine Fever Virus (CSFV) from animals vaccinated with FlagT4G as disclosed herein comprises the use of two immunoassay tests, preferably ELISAs, to differentiate three groups of individuals: animals vaccinated with FlagT4G vaccine, animals infected with CSFV and animals that are neither infected nor vaccinated.

The *in vitro* method of the present application comprises the following:
- Conducting an immunoassay, in a blood sample of the animal, for the detection of humoral response against the E2 glycoprotein of the CSFV, wherein said immunoassay is preferably a commercial ELISA for the detection of humoral response against the E2 glycoprotein; this assay will be positive in both the case of an infected animal or an animal vaccinated with FlagT4G;
- Conducting an immunoassay, in a blood sample of the animal, for the detection of humoral response against the dendrimeric peptide construct of the invention (herein named Lin₂NS3), wherein said immunoassay is preferably an ELISA; this assay will be positive in the cases where the animal is infected with CSFV but not in the cases where the animal is vaccinated with FlagT4G vaccine;

Therefore, the presently disclosed DIVA diagnostic method for the CSFV is able to differentiate animals vaccinated with FlagT4G vaccine, animals infected with CSFV and animals that are neither infected nor vaccinated, i.e. if both immunoassays are positive the animal is infected with CSFV, if the first immunoassay is negative but the second is positive the animal is vaccinated with FlagT4G and if both immunoassays are negative the animal is neither vaccinated nor infected.

In the context of the present application an "immunoassay" is understood to be any assay suitable to detect humoral response against the antigens described in the present patent application.

Even though the ELISA is the preferable immunoassay for the method of the present application, other immunoassays are also possible, such as but not limited to enzyme immunoassay (EIA), radioimmunoassay (RIA), fluoroimmunoassay (FIA), chemiluminescent immunoassay (CLIA), counting immunoassay (CIA), immunoenzymometric assay (IEMA), immunochromatographic assay, a lateral flow immunoassay, a sandwich immunoassay, an immuno-PCR assay, a proximity ligation assay, a western blot assay, or an immunoprecipitation assay.

Additional suitable immunoassays for detecting humoral response according to the method disclosed herein will be apparent to those of skill in the art.

### Methodology

### Selection of peptides

Taking into account that the CSFV FlagT4G virus contains a mutation into a highly conserved CSFV epitope within the E2 glycoprotein, the most immunogenic protein of the virus, this epitope was selected for the development of the dendrimeric constructs. In order to improve the performance of the dendrimer, this epitope was attached to a peptide within the NS3 protein, which is useful in the detection of humoral response against CSFV.

Two dendrimeric peptides based on lysine-branched constructs containing epitopes of CSFV in a 2:1 ratio were constructed:
- Lin₂NS3: 2 copies of the natural CSFV-E2 epitope TAVSPTTLRTEVVK (SEQ ID NO: 1 - letter A in Formula I) attached to one copy of the CSFV-NS3 epitope KHKVRNEVMVHWFGD (SEQ ID NO: 2 - letter B in Formula I); 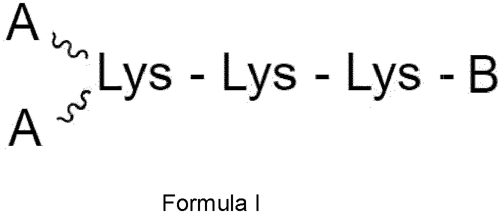
- FlagT4G₂NS3: 2 copies of the mutated CSFV FlagT4G-E2 epitope TSFNMDTLRTEVVK (SEQ ID NO 3 - letter C in Formula II) linked to one copy of the CSFV-NS3 epitope KHKVRNEVMVHWFGD (SEQ ID NO: 2 - letter B in Formula II).

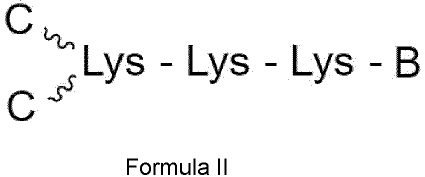

### Experimental vaccination of animals

Six-week-old pigs (Landrace x Large white, n=15), Pestivirus-free, were introduced into the biosafety level 3 animal facilities at IRTA-CReSA in Barcelona, Spain. Following a 5-day adaptation period, all the animals were inoculated with a dose of 10⁵ TCID CSFV FlagT4G virus by intramuscular injection in the right neck. Blood samples (20ml) were collected from each animal on the day of vaccination and at days 6, 13, 20 and 28 after inoculation. A second dose of 10⁵ TCID CSFV FlagT4G was administered to all the pigs by intramuscular injection in the right neck at 18 days after the first inoculation. All the animals were euthanized at 28 days after the first vaccination and serum samples were collected. The procedure for the euthanasia of the animals was based on an accepted method included in European Directive 2010/63/EU, using an anesthetic overdose of 60-100 mg of pentobarbital per kilogram of weight, administered via the vena cava.

### Evaluation of the humoral response

The specific humoral response against CSFV was evaluated in all the sera samples from the FlagT4G-vaccinated animals at different time-points using a commercial CSFV ELISA test (IDEXX), as well as with the Lin₂NS3. In the case of the FlagT4G₂NS3, only samples at 28 days after vaccination were evaluated. For the dendrimeric peptide ELISA test, a previously stablished protocol for an in-house ELISA test was performed [8,9]. Briefly, 50µL/well of a solution containing 50 µg/mL of dendrimer diluted in sodium carbonate-bicarbonate buffer (0.05 M NaHCO3, 0.05 M Na2CO3, pH 9.4) were coated overnight at 4 °C on high-binding Costar 3590 plates (Corning). After washing three times (0.05% Tween 20 in PBS), free active sites were blocked using 0.5% bovine serum albumin (BSA) in PBS during 1 h. Fifty µL/well from each serum sample (pre-diluted 1:25 in blocking buffer), were plated by duplicate and incubated at 37 °C for 1 h. After further washing, 50 µL/well of anti-swine IgG peroxidase conjugate (Sigma-Aldrich) diluted 1:20,000 in blocking buffer was added and plates were incubated at 37 °C for 1 h. The amount of coupled conjugate was determined by incubation with 50 µL/well of soluble 3,3',5,5'-tetramethylbenzidine (Calbiochem) for 20 min. at room temperature. Finally, the reaction was stopped with 50 µL/well of H₂SO₄ 1N and the absorbance was determined at 450 nm. For the commercial ELISA test, blocking percentage values above 40% were considered as positive, whereas for the dendrimeric peptide ELISA, optical density (O.D.) values above 0.4 were considered as positive. Afterwards, the capacity to detect antibody response was compared between Lin₂NS3 and FlagT4G₂NS3 using a panel of 31 serum samples from animals suffering different forms of CSF (n=25), as well as with uninfected animals (n=6).

In addition, humoral response against CSFV was assessed using Lin₂NS3 in a panel comprising over 210 serum samples, including uninfected pigs as well as animals infected with different strains of CSFV. All of these samples belonged to the serotec of the OIE reference laboratory for CSF and had been previously evaluated using the aforementioned commercial ELISA test. The number of samples, as well as the immunological status, regarding CSFV, of the animals from which the samples were obtained can be found in table 1.

**Table 1. Samples used for the assessment of CSFV antibody response by Lin₂NS3 ELISA**

| **Infection status** | **N° of animals** |
|---|---|
| Uninfected | 40 |
| Infected | 177 |
| Total | 217 |

### Results

### The Lin₂NS3 is not able to detect antibody response in animals vaccinated with FlagT4G

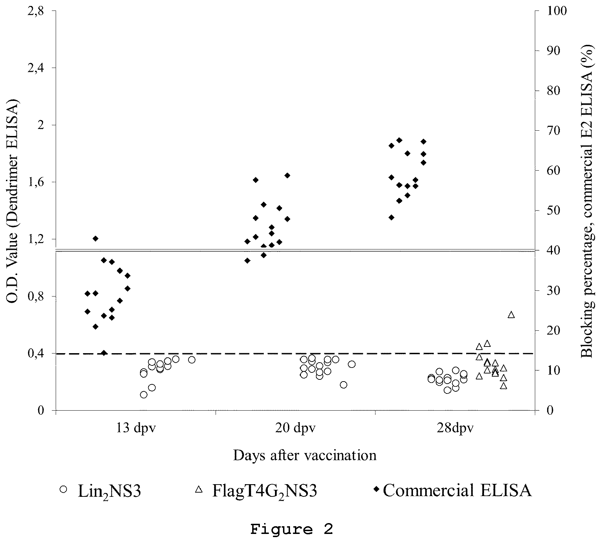

Specific anti-E2 antibody response was detected in one animal vaccinated with FlagT4G virus starting at 13 days post vaccination (dpv), using the commercial ELISA test. Afterwards, humoral response against CSFV was detected in 13 out of 15 vaccinated animals at 20 dpv and in all the vaccinated animals at 28 dpv, when samples were tested by the commercial ELISA test (Figure 2). In contrast, no antibody response was detected using the Lin₂NS3 ELISA test in samples from the vaccinated animals at any time-point. In the case of the FlagT4G₂NS3 ELISA, CSFV antibody response was detected in three animals at 28 dpv.

### Both dendrimeric constructs are able to detect antibody response in pigs infected with field CSFV strains

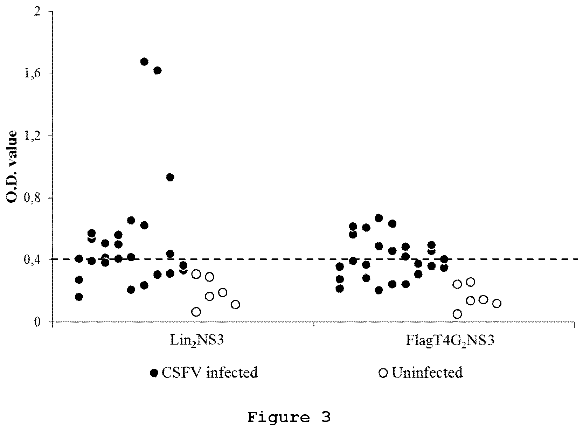

None of the serum samples from uninfected and unvaccinated pigs were positive by neither of the two dendrimer ELISA tests. Notably, in the sera from animals infected with CSFV field strains, CSFV specific antibody response was detected in 16 and 13 of the serum samples, using the Lin₂NS3 and the FlagT4G₂NS3 ELISA tests, respectively (Figure 3).

### Detection of CSFV antibody response by the Lin₂NS3 ELISA test is similar to the commercial ELISA

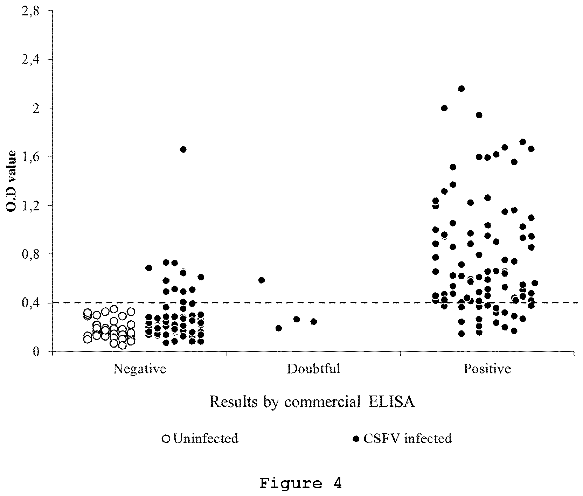

All of the serum samples from animals that had not been exposed to the virus (uninfected/unvaccinated pigs) were negative by both the commercial and dendrimeric peptide ELISA tests. Interestingly, CSFV antibody response was detected in 13 samples from CSFV infected animals using the Lin₂NS3 ELISA, which had been negative by the commercial ELISA. These samples corresponded with animals infected with a low virulence CSFV strain (Pinar del Rio) at 21 days after infection. Regarding the samples that were doubtful by the commercial ELISA test, the dendrimer technique was able to detect humoral response in a pig infected with a low virulence strain (Figure 4).

In the case of the samples that were positive by the commercial ELISA, the majority were found to be also positive for CSFV antibodies using the Lin₂NS3 test. However, 18 samples, belonging to animals after a short time post-infection (less than 15 days), were found to be negative by the Lin₂NS3 ELISA test, despite being positive by the commercial ELISA (Figure 4).

Taking into account the performance of both dendrimeric peptides (Lin₂NS3 and FlagT4G₂NS3), the inventors can confidently state that the dendrimeric peptide strategy can be a useful tool for CSFV antibody detection. Furthermore, the detection of antibodies elicited by low virulence field strains of CSFV is highly encouraging, considering that these type of infections are the most prevalent in the field nowadays and are the most likely to go unnoticed in the farms.

Considering that the FlagT4G₂NS3 ELISA was able to detect antibodies in sera from FlagT4G vaccinated animals, as well as animals infected with CSFV field strains, this test was considered not suitable for the differentiation of infected from vaccinated animals and does not comply with the DIVA concept. In contrast, the fact that the detection of antibodies against CSFV by the Lin₂NS3 dendrimeric peptide is comparable to the commercial kit (and even superior in the case of animals infected with low virulence strains), while at the same time being unable to detect the antibodies conferred by the FlagT4G vaccine, make it an ideal candidate for a DIVA companion diagnostic test for this vaccine candidate.

Several features are described hereafter that can each be used independently of one another or with any combination of the other features. However, any individual feature might not address any of the problems discussed above or might only address one of the problems discussed above. Some of the problems discussed above might not be fully addressed by any of the features described herein. Although headings are provided, information related to a particular heading, but not found in the section having that heading, may also be found elsewhere in the specification.

### Brief description of drawings

For easier understanding of this application, figures are attached in the annex that represent the preferred forms of implementation which nevertheless are not intended to limit the technique disclosed herein.

Figure 1 illustrates antigen presentation using the dendrimeric peptide strategy. Antigens can be presented using a 4:1 (A) or a 2:1 (B) ratio depending on the number of copies from each peptide that are used.

Figure 2 shows Specific Anti-CSFV antibody response in pigs after vaccination with the FlagT4G virus. Humoral response at different time-points after vaccination in terms of blocking percentage (right Y axis) and O.D. value (left Y axis) detected by commercial ELISA test (black losange) and an in-house ELISA test based on Lin₂-NS3 (white circles) or FlagT4G₂-1NS3 (white triangle). Blocking percentage values over 40% were considered as positive for the commercial ELISA test (continuous double line). For the in-house ELISA tests, O.D. values above 0.4 were determined as positive (dotted line).

Figure 3 shows CSFV antibody detection in serum samples using two different dendrimeric construct strategies. Samples from uninfected pigs (white dots) and animals infected with CSFV (black dots) were evaluated using both dendrimeric peptides. O.D. values above 0.4 were considered as positive.

Figure 4 shows the comparison between the dendrimeric ELISA strategy and the commercial test in terms of CSFV antibody detection. Serum samples from uninfected (white dots) and CSFV infected (black dots) animals were evaluated by the dendrimeric ELISA test and compared with previously obtained data from the commercial test. O.D. values above 0.4 for the Peptide ELISA (Y-axis) were considered as positive. Qualitative results of the commercial ELISA are also represented (X-axis).

### Description of embodiments

Now, preferred embodiments of the present application will be described in detail, however, these are not intended to limit the scope of this application.

The present patent application describes a dendrimeric peptide construct (Lin₂NS3) comprising two copies of the natural CSFV-E2 epitope of SEQ ID NO: 1 linked to one copy of the epitope CSFV-NS3 of SEQ ID NO: 2.
Said dendrimeric peptide construct is preferably according to formula I:

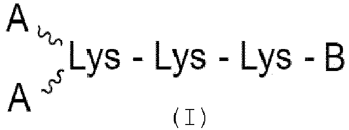

Wherein A is the CSFV-E2 epitope of SEQ ID NO: 1 and B is the CSFV-NS3 epitope of SEQ ID NO: 2.

In one embodiment, the dendrimeric peptide construct (formula I) is for use in an immunoassay for the detection of humoral response against Classical Swine Fever Virus (CSFV).

In one preferable embodiment, the dendrimeric peptide construct (formula I) is for use in an immunoassay for the detection of humoral response against CSFV as part of a method to differentiate infected animals from vaccinated animals.

In one preferable embodiment, the dendrimeric peptide construct (formula I) is for use in an immunoassay for the detection of humoral response against CSFV as part of a method to differentiate infected animals from animals vaccinated with FlagT4G.

The present patent application also describes an *in vitro* method to differentiate animals infected with Classical Swine Fever Virus (CSFV) from animals vaccinated with FlagT4G, which comprises the following steps:
- Conducting an immunoassay, in a blood sample from the animal, for the detection of humoral response against the E2 glycoprotein of the CSFV;
- Conducting an immunoassay, in a blood sample from the animal, for the detection of humoral response against the dendrimeric peptide construct of the invention (formula I);
   wherein having a positive result in the first immunoassay while having a negative result in the second immunoassay is indicative that said animal has been vaccinated with FlagT4G.

In one embodiment the immunoassay is selected from ELISA, radioimmunoprecipitation assay, immunofluorescence assay enzyme immunoassay (EIA), radioimmunoassay (RIA), fluoroimmunoassay (FIA), chemiluminescent immunoassay (CLIA), counting immunoassay (CIA), immunoenzymometric assay (IEMA), immunochromatographic assay, a lateral flow immunoassay, a sandwich immunoassay, an immuno-PCR assay, a proximity ligation assay, a western blot assay, or an immunoprecipitation assay.

In a preferable embodiment, the diagnostic method is an ELISA.

The present application also describes an ELISA kit for the differentiation of animals infected with CSFV from animals vaccinated with FlagT4G vaccine, wherein said ELISA kit comprises ELISA microplates coated with the dendrimeric peptide construct of the invention and ELISA microplates coated with the CSFV E2 glycoprotein.

In one embodiment, the ELISA kit further comprises a sample diluent solution, a washing solution, an anti-pig IgG peroxidase, a revealing solution (3,3',5,5'-Tetramethylbenzidine (TMB)) and a stop solution (H₂SO₄ 1N).

This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

### References

1. Blome, S.; Staubach, C.; Henke, J.; Carlson, J.; Beer, M. Classical swine fever-an updated review. Viruses 2017, 9, 1-24.
2. Wang, M.; Liniger, M.; Muñoz-González, S.; Bohórquez, J.A.; Hinojosa, Y.; Gerber, M.; Lopez-Soria, S.; Rosell, R.; Ruggli, N.; Ganges, L. A poly-uridine insertion in the 3'-untranslated region of classical swine fever virus activates immunity and reduces viral virulence in piglets. J. Virol. 2019**.**
3. Coronado, L.; Bohórquez, J.A.; Muñoz-González, S.; Perez, L.J.; Rosell, R.; Fonseca, O.; Delgado, L.; Perera, C.L.; Frias, M.T.; Ganges, L. Investigation of chronic and persistent classical swine fever infections under field conditions and their impact on vaccine efficacy. BMC Vet. Res. 2019, 15*.*
4. Blome, S.; Moß, C.; Reimann, I.; Konig, P.; Beer, M. Classical swine fever vaccines-State-of-the-art. Vet. Microbiol. 2017, 206, 10-20.
5. Holinka, L.G.; Fernandez-Sainz, I.; O'Donnell, V.; Prarat, M. V.; Gladue, D.P.; Lu, Z.; Risatti, G.R.; Borca, M. V. Development of a live attenuated antigenic marker classical swine fever vaccine. Virology 2009, 384, 106-113.
6. Holinka, L.G.; Fernandez-Sainz, I.; Sanford, B.; O'Donnell, V.; Gladue, D.P.; Carlson, J.; Lu, Z.; Risatti, G.R.; Borca, M. V. Development of an improved live attenuated antigenic marker CSF vaccine strain candidate with an increased genetic stability. Virology 2014, 471, 13-18.
7. Holinka, L.G.; O'Donnell, V.; Risatti, G.R.; Azzinaro, P.; Arzt, J.; Stenfeldt, C.; Velazquez-Salinas, L.; Carlson, J.; Gladue, D.P.; Borca, M. V. Early protection events in swine immunized with an experimental live attenuated classical swine fever marker vaccine, FlagT4G. PLoS One 2017, 12, e0177433.
8. Tarradas, J.; Monsó, M.; Noz, M.M.; Rosell, R.; Fraile, L.; Frias, M.T.; Domingo, M.; Andreu, D.; Sobrino, F.; Ganges, L. Partial protection against classical swine fever virus elicited by dendrimeric vaccine-candidate peptides in domestic pigs. Vaccine 2011, 29, 4422-4429.
9. Tarradas, J.; Monsó, M.; Fraile, L.; de la Torre, B.G.; Muñoz, M.; Rosell, R.; Riquelme, C.; Perez, L.J.; Nofrarías, M.; Domingo, M.; et al. A T-cell epitope on NS3 non-structural protein enhances the B and T cell responses elicited by dendrimeric constructions against CSFV in domestic pigs. Vet. Immunol. Immunopathol. 2012, 150, 36-46.
10. Lin, M.; Lin, F.; Mallory, M.; Clavijo, A. Deletions of structural glycoprotein E2 of classical swine fever virus strain alfort/187 resolve a linear epitope of monoclonal antibody WH303 and the minimal N-terminal domain essential for binding immunoglobulin G antibodies of a pig hyperimmune serum. J. Virol. 2000, 74, 11619-11625.
11. Armengol, E.; Wiesmüller, K.H.; Wienhold, D.; Büttner, M.; Pfaff, E.; Jung, G.; Saalmüller, A. Identification of T-cell epitopes in the structural and non-structural proteins of classical swine fever virus. J. Gen. Virol. 2002, 83, 551-560.

## Claims

1. A dendrimeric peptide construct of formula I: 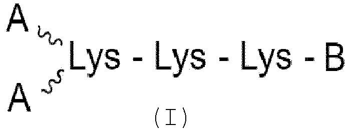 Wherein A is the CSFV-E2 epitope of SEQ ID NO: 1 and B is the CSFV-NS3 epitope of SEQ ID NO: 2.

2. The dendrimeric peptide construct of claim 1, for use in an immunoassay for the detection of humoral response against Classical Swine Fever Virus (CSFV).

3. The dendrimeric peptide construct of claim 1, for use in an immunoassay for the detection of humoral response against CSFV as part of a method to differentiate infected animals from vaccinated animals.

4. The dendrimeric peptide construct of claim 1, for use in an immunoassay for the detection of humoral response against CSFV as part of a method to differentiate infected animals from animals vaccinated with FlagT4G.

5. An *in vitro* method to differentiate animals infected with Classical Swine Fever Virus (CSFV) from animals vaccinated with FlagT4G, comprising the following steps:
- Conducting an immunoassay, in a blood sample, for the detection of humoral response against the E2 glycoprotein of the CSFV;
- Conducting an immunoassay, in a blood sample, for the detection of humoral response against the dendrimeric peptide construct of claim 1;
wherein having a positive result in the first immunoassay while having a negative result in the second immunoassay is indicative that said animal has been vaccinated with FlagT4G.

6. The method according to claim 5, wherein the immunoassay is selected from ELISA, radioimmunoprecipitation assay, immunofluorescence assay, enzyme immunoassay (EIA), radioimmunoassay (RIA), fluoroimmunoassay (FIA), chemiluminescent immunoassay (CLIA), counting immunoassay (CIA), immunoenzymometric assay (IEMA), immunochromatographic assay, a lateral flow immunoassay, a sandwich immunoassay, an immuno-PCR assay, a proximity ligation assay, a western blot assay, or an immunoprecipitation assay.

7. The method according to claim 6, wherein the immunoassay is an ELISA.

8. An ELISA kit for the differentiation of animals infected with CSFV from animals vaccinated with FlagT4G vaccine, wherein said ELISA kit comprises ELISA microplates coated with the dendrimeric peptide construct of claim 1 of the invention and ELISA microplates coated with the CSFV E2 glycoprotein.

9. The ELISA kit according to claim 8, wherein the kit further comprises a sample diluent solution, a washing solution, an anti-pig IgG peroxidase, a revealing solution (3,3',5,5'-Tetramethylbenzidine (TMB)) and a stop solution (H₂SO₄ 1N).
